# EUROPEAN PATENT APPLICATION

(11) **EP 2 077 113 A1**
(43) Date of publication of application: **08.07.2009**
(21) Application number: 08000113.4
(22) Date of filing: 04.01.2008
(51) Int. Cl.: A61K 31/47, A61K 38/16

(54) **Treatment of autoimmune diseases**

(71) Applicant: Rheinische Friedrich-Wilhelms-Universität Bonn, 53113 Bonn (DE)
(72) Inventor: Kurts, Christian, 53127 Bonn (DE); Burgdorf, Sven, 53913 Swisttal (DE)
(74) Representative: Lahrtz, Fritz

(57) **Abstract**

The present invention relates to an agent capable of preventing early endosome mediated antigen presentation for use in a method for treating or preventing an autoimmune disease or a transplant rejection. In a preferred embodiment, the agent, preferably primaquine, is capable of preventing early endosome trafficking towards the cell membrane.

## Description

The present invention relates to the treatment of an autoimmune disease or a transplant rejection.

Autoimmune diseases affect millions of people worldwide and can have devastating effects on lifespan and quality of life. Unlike most diseases where treatment involves working with the body's immune system to combat a foreign invader, in autoimmune diseases, the immune system itself is exacerbating the problem. This makes any treatment much more difficult because it must address and sometimes even combat the immune response directly to ameliorate the effects of the disease.

Despite intensive research, the scientific reasons for the onset of autoimmune diseases are still poorly understood. This is one of the reasons while current therapies of autoimmune diseases mostly focus on the relief of the symptoms of the individual disease and not on a treatment of the underlying biological mechanisms. Furthermore, strategies for preventing autoimmune diseases are rarely available.

Examples of common autoimmune diseases include early onset diabetes mellitus or multiple sclerosis.

Early onset diabetes mellitus, or Type I diabetes, is a severe, childhood, autoimmune disease, characterized by insulin deficiency that prevents normal regulation of blood glucose levels. Insulin is a peptide hormone produced by these cells within the islets of Langerhans of the pancreas. Insulin promotes glucose utilization, protein synthesis, formation and storage of neutral lipids, and is the primary source of energy for brain and muscle tissue. Type I diabetes is caused by an autoimmune reaction that results in complete destruction of these cells of the pancreas, which eliminates insulin production and eventually results in hyperglycemia and ketoacidosis.

Insulin injection therapy has been useful in preventing severe hyperglycemia and ketoacidosis, but fails to completely normalize blood glucose levels. Although insulin injection therapy has been quite successful, it does not prevent the premature vascular deterioration that is the leading cause of morbidity among diabetics today. Diabetes-related vascular deterioration, which includes both microvascular deterioration and acceleration of atherosclerosis, can eventually cause renal failure, retinal deterioration, angina pectoris, myocardial infarction, peripheral neuropathy, and atherosclerosis.

Multiple sclerosis (MS) is an autoimmune inflammatory disease of the central nervous system (CNS) caused by lymphocyte and macrophage infiltrations into the white matter resulting in demyelination. The disease is commonly observed in young Caucasian adults with Northern European ancestry. MS has been linked to the autoimmune response of T cells to myelin self-antigens. Several therapeutic approaches to MS have been previously attempted utilizing cytokines, copolymers, dimers of class II MHC-peptide complexes, peptide antigens that induce anergy, or vaccination with TCR. However, there is still no successful therapy of MS available

The present invention relates to an agent capable of preventing stable early endosome mediated antigen presentation for use in a method for treating or preventing an autoimmune disease or a transplant rejection.

Furthermore, the present invention relates to the use of an agent capable of preventing stable early endosome mediated antigen presentation for the preparation of a pharmaceutical composition for treating or preventing an autoimmune disease or a transplant rejection.

Furthermore, the present invention also relates to a method for treating or preventing an autoimmune disease, comprising administering to a patient in need thereof a therapeutically effective amount of an agent capable of preventing stable early endosome mediated antigen presentation.

Throughout the present invention, all embodiments described for the agent of the present invention also apply to the use and to the method of the present invention.

In the context of the present invention, it has been found in an in-vitro model (see the example) that in the stable early endosome MHC complexes are loaded with antigens which have been extracellularly administered and subsequently processed in the cell. These MHC-, preferably MHC I-, antigen complexes are then transported together with the early endosomes to the cellular surface, which results in the activation of effector cells, preferably cytotoxic T lymphocytes (CTLs). Furthermore, as demonstrated in the example, it has been found that an agent capable of preventing stable early endosome trafficking towards the cell membrane, namely primaquine, is capable of inhibiting the activation of cytotoxic T lymphocytes (CTLs) by dendritic cells.

Since CTLs are thought to play a major role in the onset of many autoimmune diseases or in transplant rejection (see example), it is believed in the art that their inhibition enables both the treatment of an already present autoimmune disease or transplant rejection as well as the prevention thereof. Studies in murine models of autoimmune diabetes have substantiated this postulate by showing that diabetes-prone mice whose immune system cannot activate CTL by cross-presentation are protected from diabetes (Kurts et al, 1999, Proc Natl Acad Sci USA, 96: 12703-7.; Hamilton-Williams et al, 2003, Proc Natl Acad Sci USA, 100: 6688-93).

Consequently, in view of these findings, the inventors of the present application come to the conclusion that the inhibition of stable early endosome mediated antigen presentation results in the treating or preventing of an autoimmune disease or a transplant rejection. Especially, the inventors come to the conclusion that by the administration of the agent of the invention, it is possible to treat an autoimmune disease in an early stage, namely in a stage where autoreactive CTLs are activated.

Throughout the invention, the term "stable early endosome mediated antigen presentation" means the biological mechanism that MHC complexes, especially MHC-I complexes, are loaded in the stable early endosomes with antigens. The antigens are preferably extracellular antigens uptaken by the cell which are then intracellularly processed. After the loading, the MHC-antigen complexes are transported in the membrane of the stable early endosomes to the cellular membrane, where the MHC-antigen complexes are presented to effector cells, e.g. CTLs.

According to the invention, the term "stable early endosome" (SEE) refers to that cellular compartment which is formed after internalization of an antigen or a membrane fraction and which is not transformed to a late endosome, which fuses with one or more lysosomes. This cellular compartment is further defined and explained in the example (see e.g. the section discussion). In the context of the present invention, the term "stable early endosome" and "early endosome" are used interchangeably.

Agents potentially capable of preventing stable early endosome mediated antigen presentation can be tested for this property with the help of the experimental systems described in the example.

In a preferred embodiment, the agent of the invention is an agent capable of preventing stable early endosome trafficking towards the cell membrane.

Consequently, the present invention relates preferably to an agent capable of preventing stable early endosome trafficking towards the cell membrane for use in a method for treating or preventing an autoimmune disease or a transplant rejection.

Furthermore, the present invention relates preferably to the use of an agent capable of preventing stable early endosome trafficking towards the cell membrane for the preparation of a pharmaceutical composition for treating or preventing an autoimmune disease or a transplant rejection.

Furthermore, the present invention also relates preferably to a method for treating or preventing an autoimmune disease or a transplant rejection, comprising administering to a patient in need thereof a therapeutically effective amount of an agent capable of preventing stable early endosome trafficking towards the cell membrane.

According to the invention, an agent capable of preventing stable early endosome trafficking towards the cell membrane is to be used for treating or preventing an autoimmune disease or a transplant rejection. According to the invention, the term "stable early endosome trafficking" means the direct transport of stable early endosomes to the cell membrane including the fusion of the stable early endosomes with the cell membrane without involvement of the endoplasmatic reticulum and the Golgi apparatus.

A potential agent can be tested for its property of being capable of preventing stable early endosome trafficking by using the test system described in the example where primaquine has been tested for its ability to prevent CTL activation.

In a preferred embodiment of the present invention, the agent is primaquine or a functionally active derivative thereof.

Primaquine is an 8-aminoquinoline derivative and a well-known drug used for the treatment of malaria. Its chemical name is 1,4 Pentanediamine, N⁴-(6-methoxy-8-quinolinylquinyl)-phosphate or 8-(4-amino-1-methylbutylamino)-6 methoxyquinoline diphosphate.

Functional active derivatives of primaquine as well as methods for their identification are known in the art (see e.g. Vennerstrom, J. et al., Antimicrobial Agents and Chemotherapy, March 1999, p. 598-602, Vol. 43, No. 3). Furthermore, based on the known structure of primaquine, the person skilled in the art will easily be in a position to synthesize functional active derivatives of primaquine, especially functional active derivatives which are also 8-aminoquinoline derivatives.

Other 8-aminoquinoline derivatives (and therefore primaquine derivatives) known in the art include Primaquine, Pentaquine, Pamaquine (=Plasmoquine) and Tafenoquine (the latter being available from GlaxoSmithKline).

In another preferred embodiment, the agent of the invention is capable of selectively inhibiting TAP activity in stable early endosomes.

As demonstrated in the example, it has been shown in the context of the present invention that the molecule TAP (transporter associated with antigen presentation) is an essential component of the MHC-I loading machinery in stable early endosomes. Therefore, the specific inhibition of TAP activity in stable early endosomes represents a further possibility of preventing stable early endosome mediated antigen presentation.

Such inhibitors can be tested and identified using the test systems described in the example.

According to a further preferred embodiment, the agent is a TAP inhibitor coupled to a second agent being capable of directing said TAP inhibitor to stable early endosomes.

TAP inhibitors are known in the art and include US6 (see example). Other inhibitors include UL49.5 (Koppers-Lalic et al, Proc. Natl. Acad. Sci USA, 2005, Proc Natl Acad Sci U S A. 2005, 102:5144-9) and ICP47 (Neumann et al, J Mol Biol. 1997, 272:484-92)

The function of the second agent is to direct the inhibitor to the stable early endosome. The coupling of such an inhibitor to a second agent capable of directing the inhibitor to stable early endosomes is therefore one possibility of inhibiting TAP activity in stable early endosomes. As such second agents, agents may be used which are transported to stable early endosomes, as it can be verified by co-localization experiments. One of these agents is transferrin (see example). Another known agent are ligands of the mannose receptor like mannan (see reference 15 of the example).

Consequently, in a preferred embodiment, the TAP inhibitor is US6 and the second agent is transferring or a ligand of the mannose receptor.

Further groups of agents capable of preventing stable early endosome mediated antigen presentation include agents which are able to inhibit the loading of antigens on MHC molecules, preferable MHC-I molecules, e.g. tapasin inhibitors like US3 or agents that mediate transport of MHC-I molecules into the cytosol, like US 11 (Wiertz et al, Cell, 1996, 1996 Mar 8;84(5):769-79.). Preferably, also these agents are coupled to a second gent capable of directing the agent to the stable early endosome as described above.

In a preferred embodiment of the present invention, the agent is contained in a pharmaceutical composition comprising the agent and, optionally, one or more pharmaceutically acceptable carriers.

In general, the pharmaceutical compositions of the present invention comprise a therapeutically effective amount of a therapeutic, and optionally a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated, in accordance with routine procedures, as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water or saline for injection can be provided so that the ingredients may be mixed prior to administration.

The therapeutics of the invention can be formulated as neutral or salt forms. Pharmaceutically acceptable salts include those formed with free carboxyl groups such as those derived from hydrochloric, phosphoric, acetic, oxalic, tartaric acids, etc., those formed with free amine groups such as those derived from isopropylamine, triethylamine, 2-ethylamino ethanol, histidine, procaine, etc., and those derived from sodium, potassium, ammonium, calcium, and ferric hydroxides, etc..

The amount of the therapeutic of the invention which will be effective in the treatment of a particular disorder or condition will depend on the nature of the disorder or condition, and can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from in vitro or animal model test systems. In general, suppositories may contain active ingredient in the range of 0.5% to 10% by weight; oral formulations preferably contain 10% to 95% active ingredient.

Various delivery systems are known and can be used to administer a therapeutic of the invention, e.g., encapsulation in liposomes, microparticles, and microcapsules: use of recombinant cells capable of expressing the therapeutic, use of receptor-mediated endocytosis (e.g., Wu and Wu, 1987, J. Biol. Chem. 262:4429-4432); construction of a therapeutic nucleic acid as part of a retroviral or other vector, etc. Methods of introduction include but are not limited to intradermal, intramuscular, intraperitoneal, intravenous, subcutaneous, intranasal, epidural, and oral routes. The compounds may be administered by any convenient route, for example by infusion, by bolus injection, by absorption through epithelial or mucocutaneous linings (e.g., oral, rectal and intestinal mucosa, etc.), and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compositions of the invention locally to the area in need of treatment. This may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers.

In case that the agent of the invention is primaquine or a functionally active derivative thereof, preferably formulations and administration modes are used which are known for the application of primaquine in the context of the treatment of malaria. The preferred administration is orally or intravenously, and preferred dosages are in the range of 0.1 mg/kg to 0.4 mg/kg, preferably 0.25 mg/kg.

Within the present invention, it is included that the pharmaceutical composition comprises further one or more active agents used for the treatment of autoimmune diseases like cytokines, corticosteroids, methotrexate, monoclonal antibodies, or cyclosporine / FK506.

In a preferred embodiment, pharmaceutical composition consists of the agent and one or more pharmaceutically acceptable carriers.

Throughout the present invention, it is envisaged that the agent of the invention be administered in combination with one or more other active agents used for the treatment of autoimmune diseases. These agents include insulin or cyclosporine / FK506 in the case of early onset diabetes and interferon beta or corticosteroids in the case of multiple sclerosis.

The agent of the invention may be administered before, together or after the other active agent(s).

However, it is equally preferred that said treatment consists of administering said pharmaceutical composition.

According to the invention, the active agent of the invention is used to treat or prevent an autoimmune disease.

Throughout the invention, the terms "treating" or "treatment" include the treatment of any stage of the respective disease and include a complete or partial treatment, e.g. a stabilization, of the respective disease. The terms "prevent" or "prevention" mean that the onset of the disease is prevented.

In a preferred embodiment of the present invention, the patient is a subject where, e.g. due to genetic or environmental factors, it is likely that an autoimmune disease will outbreak or a subject where early stages of an autoimmune disease, e.g. a high glucose level in the case of early onset diabetes, have already been diagnosed.

As used herein, the term "patient" refers to the recipient of a therapeutic treatment and includes all organisms within the kingdom animalia including, but not limited to animals such as cows, pigs, horses, chickens, cats, dogs, etc., and is preferably a mammal, and most preferably human.

As used herein, the term "autoimmune disease" includes "immune-related disease," "autoimmune disorder" "immunologic disorder" and "immune-related disorder." An autoimmune disease includes a disease characterized by an autoimmunization against endogenous molecules. Without being bound to any theory, it is assumed that one mechanism responsible for the development of autoimmune diseases is the so-called "cross-presentation", wherein DCs take up extracellular antigens, process them intracellularly into antigenic peptides and load them onto MHC I molecules in endosomes, possibly stable early endosomes. These complexes are then transported to the cell surface and are presented to CD8 positive T lymphocytes (CTLs) causing T cell activation, proliferation and acquisition of cytotoxic function.

Therefore, in a preferred embodiment, the autoimmune disease is an autoimmune disease induced by cross-presentation.

The autoimmune disease may be selected from the group consisting of early onset diabetes mellitus, multiple sclerosis, premature ovarian failure, sclerodermia, Sjögren's disease, lupus erythematodes, vitiligo, alopecia, polyglandular failure, Grave's disease, hypothyroidism, polymyosititis, pemphigus vulgaris, Crohn's disease, colitis ulcerosa, autoimmune hepatitis, hypopituitarism, myocardititis, Addison's disease, uveitis, pernicious anemia, hypoparathyroidism, and rheumatoid arthritis.

According to the present invention, the term "transplant rejection" means the rejection of a transplant by the recipient's immune system.

The invention is further explained by the following figures and examples, which are intended to illustrate but not limit the scope of the present invention.

### Short description of the Figures

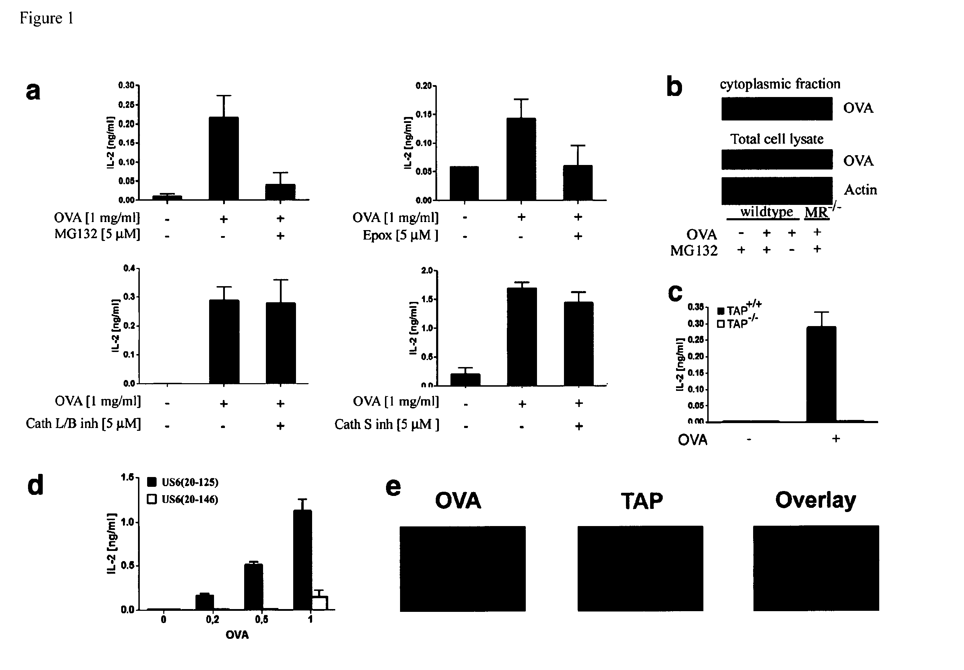

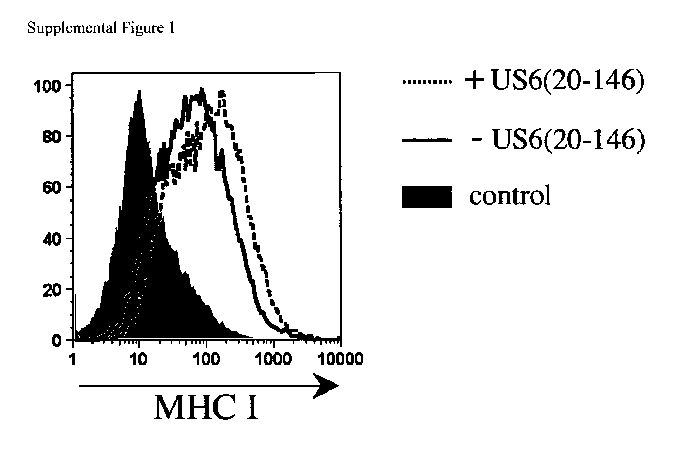

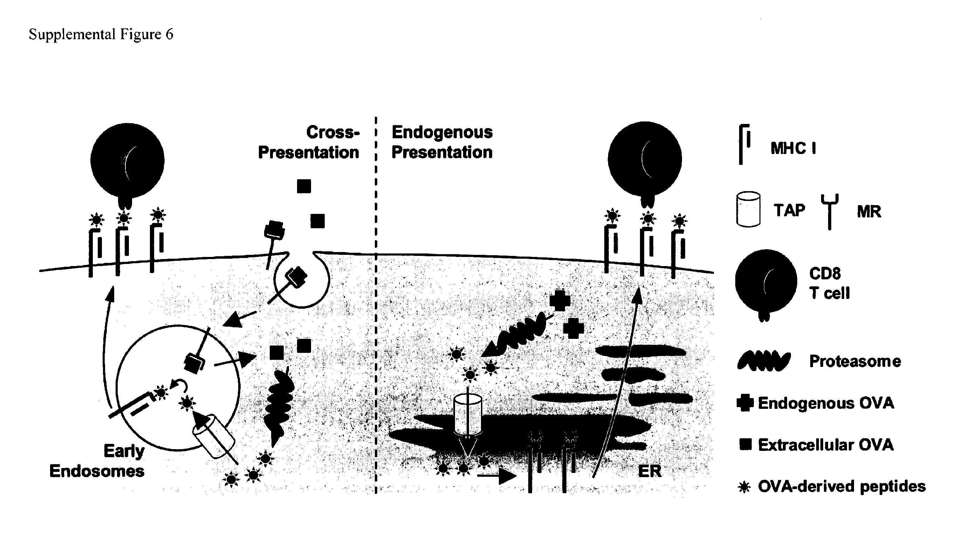

**Figure 1****.** TAP is essential for cross-presentation and is present in OVA-containing endosomes. **(a)** Cross-presentation is abolished after addition of the proteasome inhibitors MG132 and epoxomycin, but unaffected by cathepsin inhibitors. **(b)** MR-endocytosed OVA was detected in the cytosol after blocking proteasomal activity. (c) Cross-presentation is abolished in TAP-deficient DC. **(d)** Active US6(20-146) prevents cross-presentation, as opposed to inactive US6(20-125). (e) OVA-containing endosomes possess TAP. Epox: epoxomycin; cath: cathepsin.

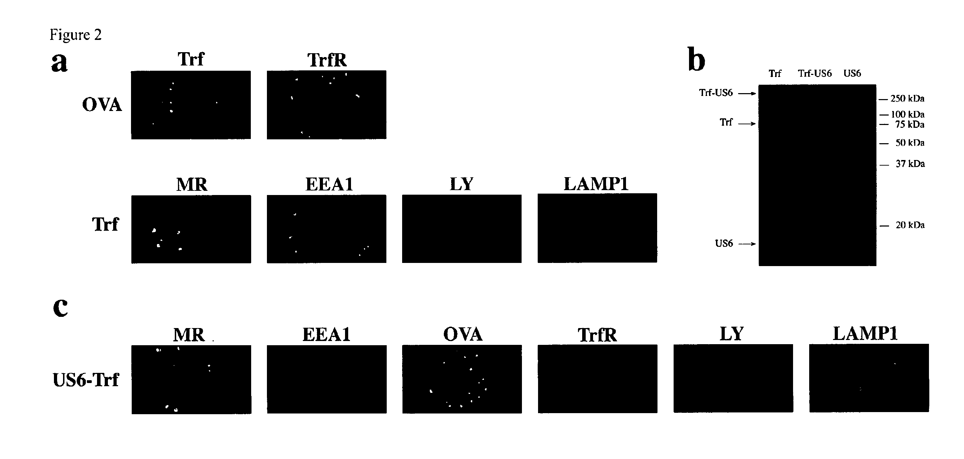

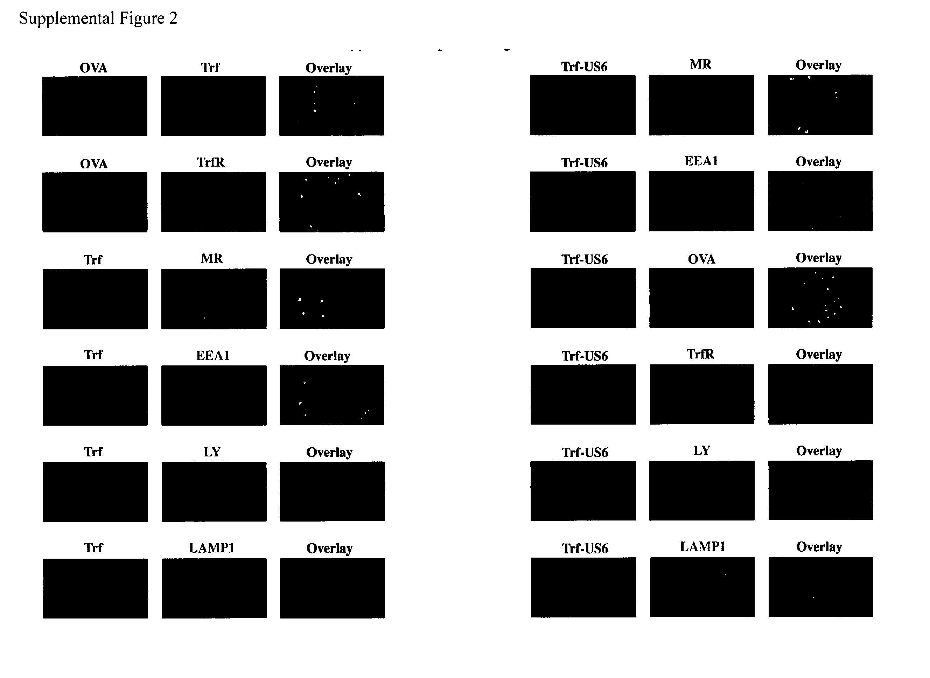

**Figure 2**. US6 linked to Trf is specifically targeted into early endosomes. **(a)** OVA is internalized into endosomes containing transferrin (Trf) and the transferrin receptor (TrfR). Trf is routed into endosomes containing the MR and EEA1, but neither pinocytosed LY, nor the lysosomal marker LAMP1. **(b)** Cross-linking US6 and Trf. **(c)** Trf-US6 is routed into early endosomes containing the MR, EEA1, OVA and the TrfR, but neither pinocytosed LY nor LAMP 1.

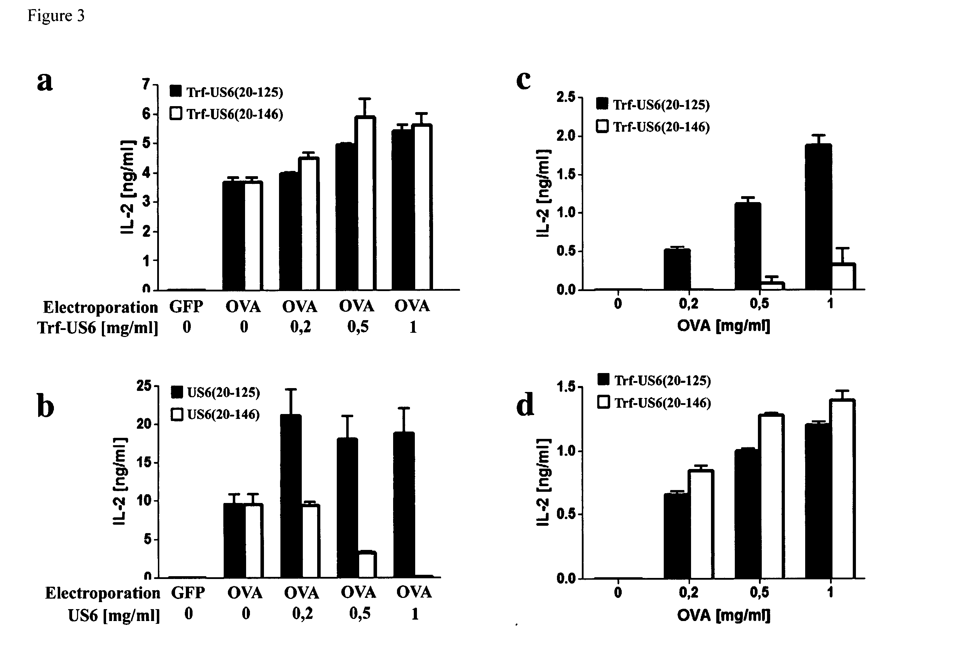

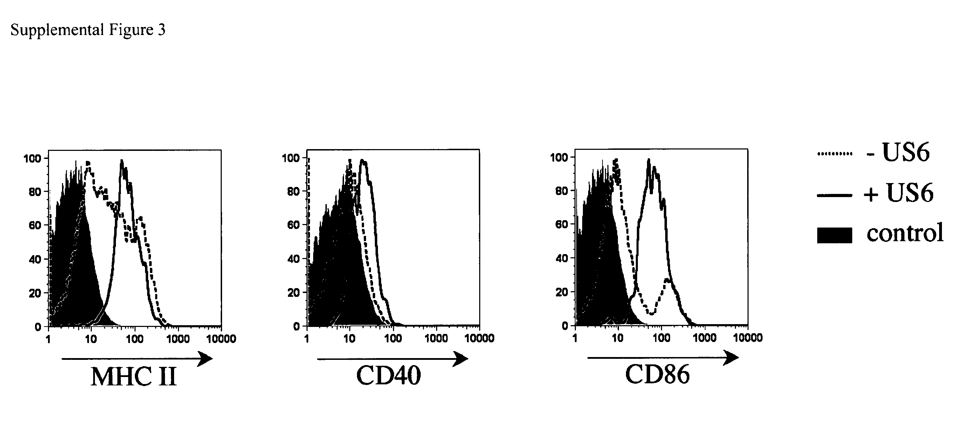

**Figure 3****.** Peptide loading for cross-presentation of soluble antigen occurs in early endosomes and not in the ER. **(a)** Trf-US6 does not affect MHC I presentation of endogenous OVA in DCs electroporated with OVA-encoding mRNA. **(b)** US6 prevents MHC I presentation of endogenous OVA. **(c)** Trf-US6 abolishes cross-presentation of OVA. **(d)** Trf-US6 does not influence MHC II-restricted presentation of extracellular OVA.

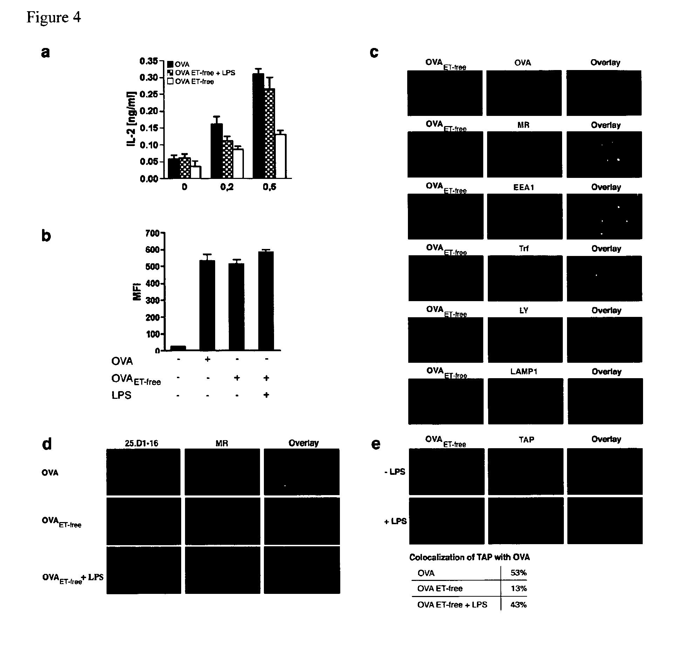

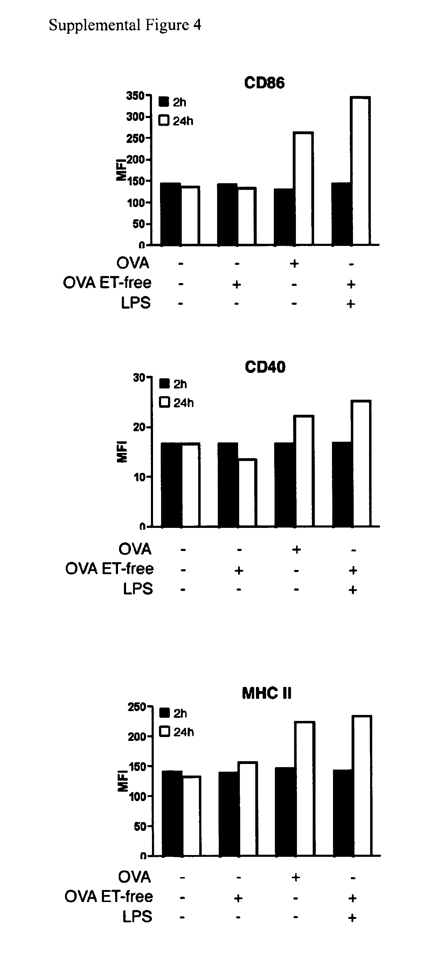

**Figure 4****.** TAP-recruitment towards early endosomes is dependent on endotoxin. (a) Cross-presentation of endotoxin-free OVA (OVA ET-free) is weak. This impairment can be partially restored by adding 10 ng/ml LPS during endocytosis. (b) DCs endocytose similar amounts of endotoxin-free OVA and ordinary OVA. (c) Ordinary and endotoxin-free OVA are routed into the same early endosomal compartment. (d) DCs use MR-endocytosed ordinary OVA, but not endotoxin-free OVA to intracellularly generate cross-presented OVA, unless 10 ng/ml LPS were present. 25.D1-16: antibody that detects cross-presented OVA. (e) TAP is only recruited to endosomes containing endotoxin-free OVA after addition of 10 ng/ml LPS. Numbers in the tables give the proportion of 100 DCs in which TAP co-localised with different forms of OVA. ET: endotoxin.

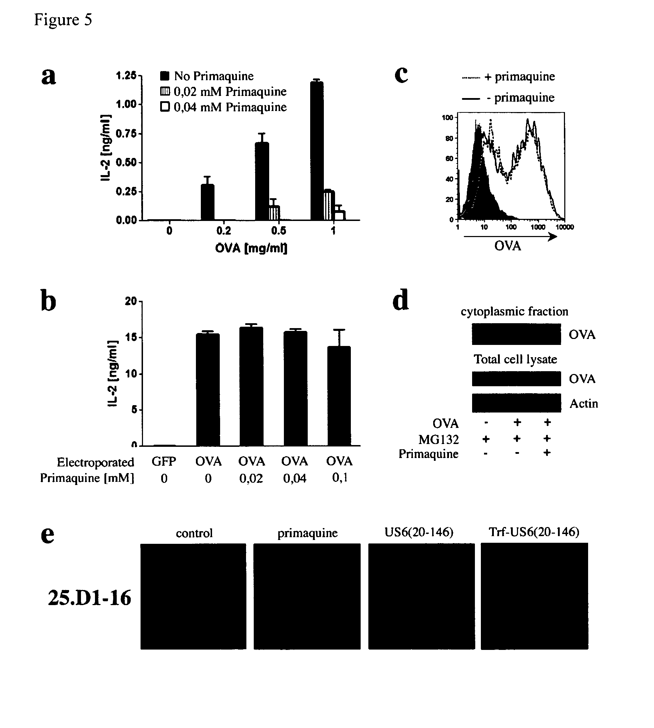

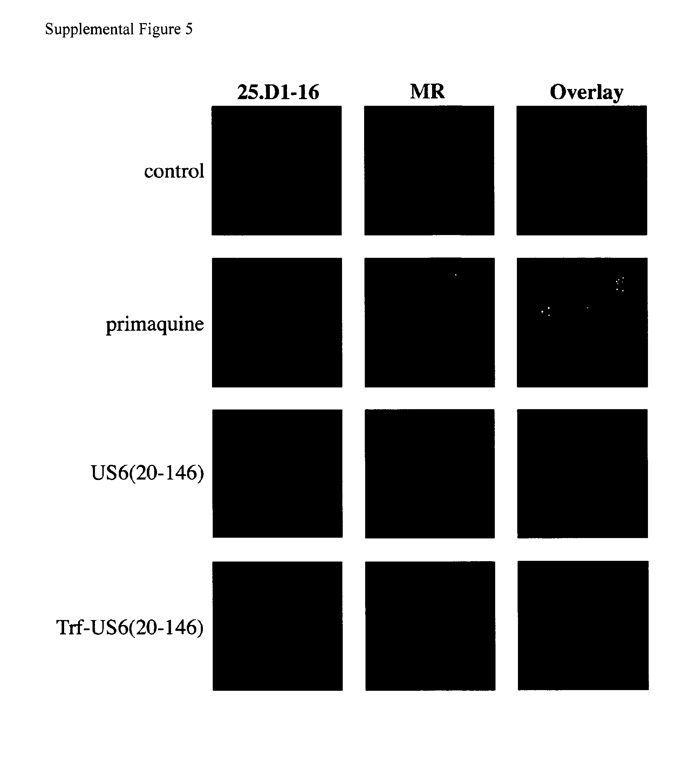

**Figure 5**. Cross-presented OVA is transported to the DC surface from endosomes, and not from the ER. **(a)** Primaquine prevents cross-presentation of OVA. **(b)** Primaquine does not affect MHC I presentation of endogenous OVA in DCs electroporated with OVA-encoding mRNA. **(c)** Primaquine does not inhibit uptake of OVA. **(d)** Primaquine does not prevent transport of OVA into the cytoplasm. **(e)** Cross-presented OVA is present in endosomes after treatment with primaquine, but not with US6 or Trf-US6.
**Supplementary** **Figure 1** MHC I expression is not downregulated after incubation with US6 for 2h. DCs were incubated with 1 mg/ml US6(20-146). After 2h, surface expression of MHC I was determined by flow cytometry.
**Supplementary** **Figure 2** Trf-US6 is routed into an early endosomal compartment. This supplemental figure adds the separate channel images to Figures 2a and 2c, which contained the overlay channel pictures only. Original TIF file has a size of 5568 x 3696 dots and a resolution of 762 dpi.
**Supplementary** **Figure 3** Incubation with recombinant US6 upregulates costimulatory molecules. DCs were incubated with 1 mg/ml US6(20-125). After 24h, surface expression of MHC II, CD40, and CD86 were determined. A clear upregulation of these molecules was observed.
**Supplementary** **Figure 4** Cell surface expression of CD86, CD40 and MHC II is unaltered after OVA treatment for 2h. Cells were incubated for 2h or 24h with OVA, endotoxin-free OVA and LPS. Cell surface expression was determined by flow cytometry and is indicated as mean florescence intensity (MFI). ET: endotoxin.
**Supplementary** **Figure 5** TAP inhibition, but not primaquine treatment, prevents the presence of cross-presented OVA in early endosomes. Cross-presented OVA (revealed by the 25.D1-16 antibody) was detectable in early endosomal compartments after treatment with primaquine, but not after treatment with US6 and Trf-US6. This supplemental figure adds a costaining with the MR to Figure 5e.
**Supplementary** **Figure 6** Spatial separation of MHC I-restricted presentation of endogenous and extracellular antigens.

### Example

The following example *inter alia* demonstrates that primaquine, is capable of inhibiting the activation of cytotoxic T lymphocytes (CTLs) by dendritic cells in an in-vitro model of cross-presentation.

### Abstract

Antiviral or antitumor immunity requires activation of cytotoxic CD8⁺ T cells by dendritic cells, which present viral or tumor antigens on MHC I molecules. The intracellular mechanisms facilitating MHC I-restricted presentation of extracellular antigens (cross-presentation) are unclear. Here, we demonstrate that cross-presentation of soluble antigen occurs in an early endosomal compartment distinct from the endoplasmic reticulum, in which endogenous antigen is loaded on MHC I. The presence of endotoxin during antigen uptake was required for recruiting TAP, an essential component of the MHC I loading machinery, towards early endosomes. Cross-presented antigen was directly transported from these endosomes in a primaquine-inhibitable fashion to the cell surface. Thus, cross-presentation is spatially and mechanistically separated from endogenous MHC I-restricted antigen presentation.

### Introduction

The mechanism of cross-presentation allows professional antigen-presenting cells (APCs), in particular dendritic cells (DCs) ^{1, 2} to induce protective immunity against intracellular microbes that do not infect DCs or against tumors that do not originate from DCs ³⁻⁵. To this end, DCs take up extracellular antigens, process them intracellularly into antigenic peptides, and load them onto major histocompatibility complex class (MHC) I molecules. These complexes are transported to the APC surface to be presented to antigen-specific CD8⁺ T lymphocytes (CTL), causing T cell activation, proliferation and acquisition of cytotoxic function against infected or malignant target cells ^{6, 7}. The cell-biological mechanisms underlying cross-presentation are not fully resolved.

Once activated, CTL survey the cells of the body for the presence of tumor or microbial antigens complexed with MHC I molecules, in order to destroy infected or malignant target cells. Such MHC I-restricted presentation of antigens synthesized within a cell (endogenous antigens) constitutively occurs in all nucleated cells of the body, in contrast to cross-presentation, which is mostly restricted to professional APCs. The intracellular mechanisms of the endogenous MHC I presentation pathway are better understood than those of cross-presentation: Endogenous antigens are degraded in the cytoplasm by the proteasome and resulting peptides are transported through the transporter associated with antigen processing (TAP) into the endoplasmic reticulum (ER), where they are loaded onto MHC I molecules ⁸. MHC I-peptide complexes are then transported via the Golgi apparatus through the secretory pathway to the cell surface for presentation to CTL.

It has been proposed that the mechanisms for endogenous MHC I presentation might be utilized by APCs also for cross-presentation ⁹. In support of this notion, cross-presentation of soluble ¹⁰⁻¹⁵ and particulate ^{10, 16, 17} antigens required the cytosolic proteasome and TAP. Phagosomes contained ER-associated molecules and components of the MHC I loading machinery such as TAP, suggesting that antigenic peptides generated by the proteasome might be re-imported through TAP directly into the phagosomes for MHC I loading there ^{10, 16, 17}. The mechanisms by which ER components reached the phagosome remain controversial ¹⁸. Furthermore, these studies were unable to discriminate between the functional requirement of TAP in the ER and phagosomal TAP, and thus could not formally distinguish between cross-presentation in the ER or in phagosomes. Also for cross-presentation of soluble antigen, direct evidence for peptide loading in the ER is lacking. Thus, the elusive compartment for cross-presentation remains to be identified.

### Methods

### Mice

MR^{-/-} mice were provided by Dr. Michel C. Nussenzweig and OT-II and OT-I.Rag-1^{-/-} mice by Dr. William R. Heath. For all experiments, mice between 8 and 16 weeks of age bred under SPF conditions were used in accordance with local animal experimentation guidelines.

### Antibodies and reagents

Rat anti-MR was from Serotec, rabbit anti-LY and all secondary antibodies from Invitrogen, rat anti-LAMP1 from Pharmingen-BD Biosciences, rabbit anti-actin from Sigma, rabbit anti-EEA1 from Dianova and goat anti-TAP from Santa Cruz Biotechnologies. Rat anti-transferrin receptor was a gift from Dr. Albert Haas. Alexa₆₄₇-labeled transferrin was from Invitrogen. US6(20-146) and US6(20-125) were prepared as described ²⁴. Endotoxin-free OVA (EndoGrade) was from Profos. All other reagents, if not specified otherwise, were from Sigma, including LPS from *E. coli.*

### Generation of bone marrow-derived DC

DCs were generated using GM-CSF as described ^{15, 19}. At day 7, CD11c⁺ cells were isolated by magnetic separation using the autoMACS^{®} system (Miltenyi). Purity of DCs was typically higher than 98%.

### Isolation and analysis of the cytoplasmic fraction of cells lysates

In order to analyse the OVA-content in the cytoplasmic fraction of cells lysates, OVA was biotinylated using the Sulfo-NHS-LC-Biotinylation kit from Perbio. DCs were incubated for 15' with biotinylated OVA [0,5 mg/ml] in the presence or absence of 0,04 mM primaquine or 5 µM MG132. Then, medium was removed and replaced with medium containing these inhibitors, but not OVA. After another 45', cytoplasmic fractions were isolated using the subcellular proteome extraction kit (Calbiochem). No endosomal or lysosomal proteins could be detected in this fraction by western blot analysis (data not shown). To concentrate OVA, cytoplasmic fractions were incubated with streptavidin-agarose for 1h, washed extensively and bound proteins were eluted for analysis by western blot using HRP-conjugated streptavidin (Biomeda).
Analysis of total cells lysates was performed after incubation for 10' on ice in lysis buffer (20 mM NaPO₄, 140 mM NaCl, 3 mM MgCl₂, 0,5% NP-40, 50 µM Leupeptin, pH 8,0).

### OT-I and OT-II activation assays

OT-I cells from OT-I rag^{-/-} mice and OT-II cells were isolated as described ^{7, 15, 19} and further purified by a CD8⁺ or CD4⁺ T cell isolation kit, respectively (Miltenyi). For cross-presentation assays, 2.5x10⁵ DCs were incubated with OVA in the presence or absence of the cathepsin S inhibitor Z-FL-COCHO [5 µM] (Calbiochem), the cathepsin L/B inhibitor Z-FF-FMK [5 µM] (Calbiochem), US6 [1 mg/ml], US6-Trf [1 mg/ml] or the proteasome inhibitors MG132 [5 µM] and epoxomicin [1 µM]. After 2h, cells were washed, fixed with 0,008% glutaraldehyde for 3' and co-incubated with 7x10⁵ OT-I or OT-II cells. IL-2 concentrations in the supernatant were determined after 18h by ELISA.
To investigate the effect of primaquine on cross-presentation, cells were incubated with OVA and primaquine for 10'. Then, medium was removed and replaced with medium containing primaquine but no OVA for additional 110'. Afterwards, cells were fixed and cocultured with OT-I cells as described above.
For the MHC I-restricted presentation of endogenous antigens, OVA-encoding mRNA was synthesized using the T7 mMessage mMachine Ultra Kit (Ambion). Electroporation of mRNA was performed as described ^{15, 44}, and typically yielded efficiencies of about 80%. After electroporation, cells were cultured 2h in the presence or absence of indicated concentrations of primaquine, US6 or Trf-US6, then fixed and co-cultured with OT-I cells as described above.

### Preparation of fluorescent OVA and uptake experiments

Soluble OVA was rendered fluorescent by chemical conjugation to alexa₆₄₇ (Invitrogen). The labeling procedure involved gel-filtration for removing of low molecular weight molecules such as peptide fragments or unbound fluorochrome. Uptake experiments were performed as described ¹⁵.

### Cross-linking of Trf and US6

Coupling experiments were performed using heterobifunctional crosslinker reagents from Perbio according to manufacturers' instructions. Briefly, maleimid groups were coupled to 1 mg US6 by incubation for 30' at room temperature with 19 µl sulfosuccinimidyl 4-[N-maleimidomethyl]cyclohexane-1-carboxylate solution. Sulfhydryl groups were coupled to 1 mg Trf by incubation with 0,2 mg N-succinimidyl S-acetylthioacetate for 30', followed by reduction achieved by adding 10% hydroxylamine solution (0,62 M hydroxylamine hydrochloride; 25 mM EDTA, pH 7.4) for 2h. Afterwards, modified US6 and Trf were coupled by co-incubation for 30' in a molar ratio US6:Trf of 5:1. After coupling, free US6 was removed by extensive washing, using a vivaspin column with a molecular cutoff of 30 kDa (Sartorius).
For coupling of alexa₆₄₇-labeled US6 to Trf, 1 mg US6 was coupled to alexa₆₄₇ (Invitrogen) in the presence of 0,336 mg of the heterobifunctional crosslinking reagent succinimidyl 4-formylbenzoate (SFB). After 30', free alexa₆₄₇ and SFB were removed by gel filtration. In parallel, 1 mg Trf was conjugated to 75 µg succinimidyl 4-hydrazinonicotinate acetone hydrazone. Afterwards, alexa₆₄₇-labeled US6 was coupled to Trf and free US6 was removed as described above.

### Immunofluoreseence

Cells were pulsed with fluorochrome-labeled OVA [10 µg/ml], LY [0,3 mg/ml], US6 [10 µg/ml], Trf [10 µg/ml] or US6-Trf [10 µg/ml] for 15' and chased with medium for another 15'. Staining experiments, including the 25.D1-16 staining, were performed as described ¹⁵. Nuclei were revealed with 1 µg/ml DAPI. Cells were analyzed with a IX71 microscope using analySIS software (Olympus).

### Flow-cytometry

Flow-cytometry was performed on an LSR® (BD-Biosciences). Data were analyzed using Flow-Jo® software (Tristar).

### Results

### TAP is essential for cross-presentation and is present in OVA-containing endosomes

We have recently reported that distinct antigen uptake mechanisms dictate whether endocytosed antigen is cross-presented to CTL or presented on MHC II molecules to CD4⁺ T helper cells ^{15, 19}. In these studies, DCs exclusively used the mannose receptor (MR) to take up the widely employed model antigen, soluble ovalbumin (OVA), into distinct early endosomal compartments specialized for cross-presentation ^{15, 19}. The resulting activation of OVA-specific CTL (OT-I cells) could be blocked by MG132 and epoxomycin, two proteasome inhibitors (Fig. 1a), consistent with previous studies reporting the requirement of the proteasome for cross-presentation ^{11, 14, 16, 17, 19}. In contrast, OT-I cell activation was unaltered by inhibitors of cathepsins (Fig. 1a), which degrade proteins within endosomes ²⁰, suggesting that soluble antigen must leave early endosomes to reach the proteasome, as previously proposed ^{11, 21, 22}. This indeed was the case, as we could detect the presence of MR-endocytosed OVA in the cytosol, if the proteasome was prevented from instantly degrading it after export from early endosomes (Fig. 1b).

Cross-presentation of OVA has been reported to be TAP-dependent ^{9, 10, 13, 14}. Also in our system, cross-presentation of MR-endocytosed OVA required TAP, since DCs from TAP-deficient mice were incapable of OT-I cell activation (Fig. 1c). However, it has been shown that TAP-deficient cells express ineffectual surface MHC I ²³, which also might explain the inability of such cells to activate CTL. Hence, we used the recombinant protein US6, a low-molecular weight luminal TAP inhibitor of the cytomegalovirus ²⁴. Consistent with previous studies by others ^{10, 12}, we observed that a recombinant US6 fragment (amino acids 20-146) containing the TAP-inhibiting domain completely abolished cross-presentation of MR-endocytosed OVA by TAP-competent DCs, as opposed to a further truncated inactive fragment US6(20-125) (Fig. 1d). This inhibition of cross-presentation was not due to low MHC I levels, because DCs in our system were fixed after 2h of culture with OVA, and MHC I levels were not significantly altered by US6 within this short time frame (Supplementary Fig. 1).

Since it has been demonstrated that OVA-transporting endosomes contained some ER components such as calnexin ^{10, 13}, we investigated whether endosomes containing MR-endocytosed OVA possessed also TAP, as reported for phagosomes carrying cell-associated antigen for cross-presentation ^{10, 16, 17}. Indeed, immunofluorescence staining for TAP showed a clear colocalization with endocytosed OVA (Fig. 1e). In addition, we noted perinuclear TAP staining that colocalized with calnexin, a frequently used ER marker (data not shown). These findings suggested that not only the ER, but also the early endosomes carrying antigen for cross-presentation possessed this transporter, raising the possibility that proteasome-generated peptides might be imported into early endosomes and loaded there on MHC I molecules.

### Proteasome-generated antigenic peptides are re-imported by TAP into early endosomes for cross-presentation on MHC I molecules

To investigate whether endosomal TAP was involved in cross-presentation of OVA, an approach was required to selectively deliver the inhibitor US6 into early endosomes. This was achieved by using transferrin as a "trojan horse". When this molecule has bound iron, it is taken up by the transferrin receptor into recycling endosomes, in which it detaches from its receptor, releases its cargo and recycles back to the cell surface ²⁵. Importantly, in DCs, transferrin and its receptor strictly co-localized with MR-endocytosed OVA, the MR and the early endosomal marker EEA1 (Fig. 2a, separate channel images in Supplementary Fig. 2), indicating that transferrin traffics through the early endosomes dedicated to cross-presentation. Transferrin co-localized neither with lucifer yellow (LY), a substance which is taken up by fluid phase pinocytosis and is rapidly targeted towards lysosomal compartments ¹⁵, nor with the lysosomal marker LAMP-1, demonstrating exclusion from the late endosomal/lysosomal MHC II-loading compartment. We reasoned that this intracellular routing pattern may allow selective introduction of US6 into early endosomes. Thus, we chemically linked this inhibitor to transferrin, resulting in complexes (Trf-US6) with a molecular size of above 250 kDa (Fig. 2b). Trf-US6 was targeted as intended, as evident from its co-localization with the transferrin receptor, the MR, MR-endocytosed OVA and EEA1, but not with LY or LAMP1 (Fig. 2c, separate channel images in Supplemetary Fig. 2). The absence of Trf-US6 routing into the ER was confirmed using DCs that had been transfected with OVA-encoding mRNA, resulting in endogenous expression of this antigen. In these DCs, Trf-US6(20-146) did not inhibit OT-I cell activation (Fig. 3a), demonstrating that it did not inhibit TAP in the ER. In contrast, OT-I cell activation by endogenous OVA was abrogated by the active US6(20-146) small molecular weight protein (Fig. 3b), which is known to block endogenous ¹², as well as cross-presentation of OVA ¹⁰. Importantly, Trf-US6(20-146) indeed could inhibit cross-presentation, as opposed to inactive Trf-US6(20-125) (Fig. 3c). These findings confirmed the presence of endosomal TAP and demonstrated that it was required to mediate access of OVA-derived peptides into early endosomes for cross-presentation. Activation of OVA-specific CD4⁺ T cells (OT-II cells) was unaffected by Trf-US6, excluding toxic effects and demonstrating specificity of inhibition for MHC I-restricted presentation of exogenous antigens (Fig. 3d). We noted that addition of inactive US6(20-125) increased OT-I cell activation by endogenous OVA (Fig. 3b). This may have been due to endotoxins resulting from its production in recombinant bacteria, as LPS treatment has been shown to stimulate proteasomal and TAP activity in DCs ²⁶. In support of this interpretation, we noted upregulation of costimulatory molecules by DCs treated with US6 (Supplementary Fig. 3).

### Endotoxin causes recruitment of TAP to early endosomes and cross-presentation in this compartment

Immunity requires DC maturation, induced for example by microbial endotoxins such as LPS, which increases expression of co-stimulatory molecules on the DC surface ^{27, 28}. Work on MHC II-restricted antigen presentation has demonstrated that these agents increase also lysosomal antigen processing by several mechanisms ²⁹⁻³³. There is evidence that microbial molecular patterns enhance the intracellular mechanisms also of cross-presentation ^{26, 34, 35}. Since pathogen-derived antigens as well as commonly used model antigens such as ordinary OVA often contain traces of endotoxins ^{36, 37}, we investigated the influence of endotoxin on cross-presentation of MR-endocytosed OVA. We noted that endotoxin-free OVA was cross-presented far less efficiently than ordinary OVA (Fig. 4a), and that this could be partially restored by adding LPS (Fig. 4a). Impaired cross-presentation was not due to reduced antigen uptake, since both endotoxin-free and ordinary OVA were internalized by DCs to a similar extent (Fig. 4b). It was not due to a requirement of endotoxins for intracellular antigen routing, since ordinary OVA and endotoxin-free OVA trafficked through Trf⁺ EEA1⁺ MR⁺ early endosomes, but not through LAMP1⁺ late endosomes/lysosomes like pinocytosed LY did (Fig. 4c). Finally, reduced cross-presentation was not due to a lack of expression of co-stimulatory molecules, as in our system DCs were fixed 2h after culture with OVA, and within this time frame no altered expression of these molecules was observed (Supplemental Fig. 4). Therefore, we speculated that the stimulatory effect of endotoxin might instead result from increased activity of the cross-presentation machinery. To investigate this hypothesis, we first determined the influence of endotoxin on the generation of cross-presented OVA by immunofluorescence microscopy, using the 25-D1.16 antibody, which specifically recognizes the OVA-peptide SIINFEKL bound to the MHC I molecule K^{b 38}. Using this technique we had previously demonstrated cross-presented OVA in early endosomes, where it co-localised with MR-endocytosed ordinary OVA ¹⁵. However, when we employed endotoxin-free OVA, which was cross-presented much less efficiently (Fig. 4a), no cross-presented OVA was detectable in early endosomes using the 25-D1.16 antibody (Fig. 4d). Addition of LPS restored generation of cross-presented OVA in early endosomes (Fig. 4d), consistent with the increased activation of OT-I cells under these conditions (Fig. 4a). Since endosomal TAP was crucial for cross-presentation of OVA (Fig. 3c), we then investigated whether the stimulatory effect of endotoxins was linked to the recruitment of TAP towards OVA-containing endosomes. Indeed, TAP co-localized with endocytosed ordinary OVA (Fig. 1e), but not with internalized endotoxin-free OVA (Fig. 4e). Again this deficiency could be overcome by exogenously adding LPS, in whose presence TAP was detected in endosomes carrying endotoxin-free OVA (Fig. 4e). Taken together, these findings demonstrate that endotoxin was required for recruitment of TAP to early endosomal compartments and for the generation of cross-presented OVA therein.

### Cross-presented OVA is transported to the DC surface from endosomes.

The inhibition of cross-presentation after specifically blocking endosomal TAP demonstrated that antigenic peptides had to enter early endosomes in order to be complexed with MHC I molecules (Fig. 4c). Next, we investigated whether these complexes were directly transported from early endosomes to the cell surface. To this end, we used the drug primaquine that prevents endosome trafficking towards the cell membrane ³⁹. Primaquine indeed prevented OT-I cell activation by cross-presentation in a dose-dependent manner (Fig. 5a), demonstrating that cross-presented OVA on the cell surface originated from endosomes. In contrast, it did not affect OT-I cell activation by DCs transfected with OVA-encoding mRNA (Fig. 5b), confirming that the classical endogenous MHC I presentation pathway through the ER and the Golgi apparatus was not compromised by this drug. Primaquine neither affected OVA uptake (Fig 5c), nor its export from endosomes to the cytoplasm (Fig. 5d), ruling out that it inhibited other steps of cross-presentation. Cross-presented OVA, revealed by the 25.D1-16 antibody, remained detectable in early endosomes despite primaquine treatment (Fig. 5e, Supplementary Fig.5), confirming that this drug did not abrogate the generation, but instead prevented transport of cross-presented OVA to the cell surface. In contrast, US6- and Trf-US6-treated DCs showed no 25-D1.16 staining (Fig. 5e, Supplementary Fig. 5), once more demonstrating that endosomal TAP activity was required for generation of MHC I/peptide complexes for cross-presentation.

### Discussion

In this study, we provide formal experimental evidence that cross-presentation is spatially separated from endogenous MHC I-restricted antigen presentation in DCs. Our findings support the following mechanism for cross-presentation of soluble antigen (Supplemental Fig. 6): OVA was taken up by the MR into stable early endosomes ¹⁵, was exported into the cytoplasm (Fig. 1b), processed by the proteasome (Fig. 1a), and re-imported via TAP into the same endosomal compartment (Fig. 3c, 5e) for loading on MHC I molecules. This explains our previous observation that MR-endocytosed OVA and cross-presented OVA co-localized only in early endosomes ¹⁵. From these endosomes, cross-presented OVA was transported directly to the cell membrane for presentation to CTL (Fig. 5a). TAP-mediated import into the ER was required only for endogenous presentation, but not for cross-presentation.

This scenario (Supplemental Fig. 6) is reminiscent of a model for cross-presentation of phagocytosed particulate antigens, which required antigen export from phagosomes towards the cytoplasmic proteasome, followed by TAP-mediated transport of antigen-derived peptides ^{10, 16, 17}. Although TAP was detected in the phagosomal membrane, formal experimental evidence for a functional role of phagosomal TAP was not reported. Such evidence may be attained by specifically targeting the TAP-inhibitor US6 into phagosomes, using a similar approach as described here.

In a previous study examining cross-presentation of soluble antigens, Ackerman *et al* reported that externally added β2-microglobulin, a small component of MHC I, was targeted to the ER by unknown pathways ¹². Based on this observation, it was proposed that a mechanism that can introduce extracellular proteins into the ER may facilitate cross-presentation, by utilizing the presentation mechanisms for endogenous antigen ¹². However, in those studies, extracellular OVA was found to be targeted to "ER-like structures", which denoted vesicles spatially distinct from the perinuclear ER yet containing certain ER-components such as calnexin ¹⁰. As extracellular proteins like OVA are usually internalized into endosomes, direct access of antigen to the ER is probably restricted to a subset of antigens.

The present study is the first to provide several lines of evidence that endosomes carrying soluble antigens for cross-presentation contained TAP and that its function was essential for cross-presentation (Fig. 1e, 3c, 4e, 5e). The endosomal content of TAP depended on the presence of endotoxin during endocytosis. Endotoxin-dependent TAP recruitment to early endosomes may represent a mechanism by which efficient cross-presentation can be restricted to microbial antigens that carry endotoxin or other pathogen-associated molecular patterns. Future studies may elucidate whether further stimuli exist that can induce such recruitment, and whether it occurs by vesicular fusion or, as discussed for particulate antigens, by ER-mediated endocytosis ^{10, 16-18, 34, 40}.

These findings imply that the use of highly pure, endotoxin-free antigens would produce only weak cross-presentation responses, as observed here for endotoxin-free OVA. As this is the case for most autoantigens, our present findings may explain why *in vivo* cross-presentation of a transgenic self antigen occurred only when it was expressed at high doses ⁴¹.

The recruitment of TAP to the organelles of cross-presentation represents a further mechanism by which endotoxins can stimulate immunity. For MHC II-restricted antigen presentation, endotoxins have been shown to increase CD4⁺ T cells activation by a variety of mechanisms beyond mere upregulation of co-stimulatory molecules ^{27, 28}, including stimulatory effects on phagocytosis and phagosome maturation ⁴², lysosomal antigen processing ³², loading of processed antigens onto MHC II molecules ³³, and routing and stabilization of MHC II/peptide complexes on the DC surface ^{29-31, 43}. The effect of endotoxin on cross-presentation is less clear. Our finding that it improved cross-presentation by relocating TAP towards early endosomes and thereby permitting entry of antigenic peptides differs from the situation in MHC II-restricted presentation of extracellular antigen, where peptides were generated within the competent organelles and endotoxins operated by enabling their loading on MHC II molecules ³³. The recruitment of TAP as an essential component of the cross-presentation machinery may be complemented by other stimulatory effects of endotoxin on DCs, such as increasing the activity of TAP and the proteasome ²⁶, or upregulation of co-stimulatory molecules.

The spatial separation of MHC I-restricted antigen processing for endogenous and extracellular antigens may ensure that peptides from extracellular antigens do not have to compete with the large pool of endogenous peptides for MHC I molecules in the ER. Such separation may hinder viruses that inhibit endogenous presentation in infected APCs from compromising cross-presentation. Furthermore, distinct loading sites theoretically may allow distinct proteasomal subsets to selectively process endogenous and extracellular antigens, or permit the development of drugs that selectively inhibit one of these processes, such as primaquine did. Finally, our findings pertain to the development of therapeutic vaccines against viruses or tumors, by revealing the intracellular compartments in which a vaccine is processed when pathways such as MR-mediated endocytosis are used.

### References

1. den Haan, J.M., Lehar, S.M. & Bevan, M.J. CD8(+) but not CD8(-) dendritic cells cross-prime cytotoxic T cells in vivo. J Exp Med 192, 1685-1696 (2000).
2. Kurts, C., Cannarile, M., Klebba, I. & Brocker, T. Dendritic cells are sufficient to cross-present self-antigens to CD8 T cells in vivo. J Immunol 166, 1439-1442 (2001).
3. Huang, A.Y. et al. Role of bone marrow-derived cells in presenting MHC class I-restricted tumor antigens. Science 264, 961-965 (1994).
4. Sigal, L.J., Crotty, S., Andino, R. & Rock, K.L. Cytotoxic T-cell immunity to virus-infected non-haematopoietic cells requires presentation of exogenous antigen. Nature 398, 77-80 (1999).
5. Villadangos, J.A., Heath, W.R. & Carbone, F.R. Outside looking in: the inner workings of the cross-presentation pathway within dendritic cells. Trends Immunol 28, 45-47 (2007).
6. Bevan, M.J. Cross-priming for a secondary cytotoxic response to minor H antigens with H-2 congenic cells which do not cross-react in the cytotoxic assay. J Exp Med 143, 1283-1288 (1976).
7. Kurts, C. et al. Constitutive class I-restricted exogenous presentation of self antigens in vivo. J Exp Med 184, 923-930 (1996).
8. Abele, R. & Tampe, R. The ABCs of immunology: structure and function of TAP, the transporter associated with antigen processing. Physiology 19, 216-224 (2004).
9. Ackerman, A.L. & Cresswell, P. Cellular mechanisms governing cross-presentation of exogenous antigens. Nat Immunol 5, 678-684 (2004).
10. Ackerman, A.L., Kyritsis, C., Tampe, R. & Cresswell, P. Early phagosomes in dendritic cells form a cellular compartment sufficient for cross presentation of exogenous antigens. Proc Natl Acad Sci U S A 100, 12889-12894 (2003).
11. Norbury, C.C. et al. CD8+ T cell cross-priming via transfer of proteasome substrates. Science 304, 1318-1321 (2004).
12. Ackerman, A.L., Kyritsis, C., Tampe, R. & Cresswell, P. Access of soluble antigens to the endoplasmic reticulum can explain cross-presentation by dendritic cells. Nat Immunol 6, 107-113 (2005).
13. Imai, J., Hasegawa, H., Maruya, M., Koyasu, S. & Yahara, I. Exogenous antigens are processed through the endoplasmic reticulum-associated degradation (ERAD) in cross-presentation by dendritic cells. Int Immunol 17, 45-53 (2005).
14. Ackerman, A.L., Giodini, A. & Cresswell, P. A role for the endoplasmic reticulum protein retrotranslocation machinery during crosspresentation by dendritic cells. Immunity 25, 607-617 (2006).
15. Burgdorf, S., Kautz, A., Bohnert, V., Knolle, P.A. & Kurts, C. Distinct pathways of antigen uptake and intracellular routing in CD4 and CD8 T cell activation. Science 316, 612-616 (2007).
16. Houde, M. et al. Phagosomes are competent organelles for antigen cross-presentation. Nature 425, 402-406 (2003).
17. Guermonprez, P. et al. ER-phagosome fusion defines an MHC class I cross-presentation compartment in dendritic cells. Nature 425, 397-402 (2003).
18. Touret, N. et al. Quantitative and dynamic assessment of the contribution of the ER to phagosome formation. Cell 123, 157-170 (2005).
19. Burgdorf, S., Lukacs-Kornek, V. & Kurts, C. The mannose receptor mediates uptake of soluble but not of cell-associated antigen for cross-presentation. J Immunol 176, 6770-6776 (2006).
20. Riese, R.J. & Chapman, H.A. Cathepsins and compartmentalization in antigen presentation. Curr Opin Immunol 12, 107-113 (2000).
21. Rodriguez, A., Regnault, A., Kleijmeer, M., Ricciardi-Castagnoli, P. & Amigorena, S. Selective transport of internalized antigens to the cytosol for MHC class I presentation in dendritic cells. Nat Cell Biol 1, 362-368 (1999).
22. Hotta, C., Fujimaki, H., Yoshinari, M., Nakazawa, M. & Minami, M. The delivery of an antigen from the endocytic compartment into the cytosol for cross-presentation is restricted to early immature dendritic cells. Immunology 117, 97-107 (2006).
23. Song, R., Porgador, A. & Harding, C.V. Peptide-receptive class I major histocompatibility complex molecules on TAP-deficient and wild-type cells and their roles in the processing of exogenous antigens. Immunology 97, 316-324 (1999).
24. Kyritsis, C. et al. Molecular mechanism and structural aspects of transporter associated with antigen processing inhibition by the cytomegalovirus protein US6. J Biol Chem 276, 48031-48039 (2001).
25. Ciechanover, A., Schwartz, A.L. & Lodish, H.F. Sorting and recycling of cell surface receptors and endocytosed ligands: the asialoglycoprotein and transferrin receptors. J Cell Biochem 23, 107-130 (1983).
26. Gil-Torregrosa, B.C. et al. Control of cross-presentation during dendritic cell maturation. Eur J Immunol 34, 398-407 (2004).
27. Bancherau, J. & Steinman, R.M. Dendritic cells and the control of immunity. Nature 392, 245-252 (1998).
28. Mellman, I. & Steinman, R.M. Dendritic cells: specialized and regulated antigen processing machines. Cell 106, 255-258 (2001).
29. Cella, M., Engering, A., Pinet, V., Pieters, J. & Lanzavecchia, A. Inflammatory stimuli induce accumulation of MHC class II complexes on dendritic cells. Nature 388, 782-787 (1997).
30. Turley, S.J. et al. Transport of peptide-MHC class II complexes in developing dendritic cells. Science 288, 522-527 (2000).
31. Pierre, P. et al. Developmental regulation of MHC class II transport in mouse dendritic cells. Nature 388, 787-792 (1997).
32. Trombetta, E.S., Ebersold, M., Garrett, W., Pypaert, M. & Mellman, I. Activation of lysosomal function during dendritic cell maturation. Science 299, 1400-1403 (2003).
33. Blander, J.M. & Medzhitov, R. Toll-dependent selection of microbial antigens for presentation by dendritic cells. Nature 440, 808-812 (2006).
34. Maurer, T. et al. CpG-DNA aided cross-presentation of soluble antigens by dendritic cells. Eur J Immunol 32, 2356-2364 (2002).
35. Schulz, O. et al. Toll-like receptor 3 promotes cross-priming to virus-infected cells. Nature 433, 887-892 (2005).
36. Kopp, E. & Medzhitov, R. Recognition of microbial infection by Toll-like receptors. Curr Opin Immunol 15, 396-401 (2003).
37. Watanabe, J., Miyazaki, Y., Zimmerman, G.A., Albertine, K.H. & McIntyre, T.M. Endotoxin contamination of ovalbumin suppresses murine immunologic responses and development of airway hyper-reactivity. J Biol Chem 278, 42361-42368 (2003).
38. Porgador, A., Yewdell, J.W., Deng, Y., Bennink, J.R. & Germain, R.N. Localization, quantitation, and in situ detection of specific peptide-MHC class I complexes using a monoclonal antibody. Immunity 6, 715-726 (1997).
39. van Weert, A.W., Geuze, H.J., Groothuis, B. & Stoorvogel, W. Primaquine interferes with membrane recycling from endosomes to the plasma membrane through a direct interaction with endosomes which does not involve neutralisation of endosomal pH nor osmotic swelling of endosomes. Eur J Cell Biol 79, 394-399 (2000).
40. Gagnon, E. et al. Endoplasmic reticulum-mediated phagocytosis is a mechanism of entry into macrophages. Cell 110, 119-131 (2002).
41. Kurts, C., Miller, J.F., Subramaniam, R.M., Carbone, F.R. & Heath, W.R. Major histocompatibility complex class I-restricted cross-presentation is biased towards high dose antigens and those released during cellular destruction. J Exp Med 188, 409-414 (1998).
42. Blander, J.M. & Medzhitov, R. Regulation of phagosome maturation by signals from toll-like receptors. Science 304, 1014-1018 (2004).
43. Shin, J.S. et al. Surface expression of MHC class II in dendritic cells is controlled by regulated ubiquitination. Nature 444, 115-118 (2006).
44. Van Meirvenne, S. et al. Efficient genetic modification of murine dendritic cells by electroporation with mRNA. Cancer Gene Ther 9, 787-797 (2002).

## Claims

1. An agent capable of preventing stable early endosome mediated antigen presentation for use in a method for treating or preventing an autoimmune disease or a transplant rejection.

2. The agent of claim 1, wherein the agent is capable of preventing stable early endosome trafficking towards the cell membrane.

3. The agent of claim 2, wherein the agent is primaquine or a functionally active derivative thereof.

4. The agent of claim 1, wherein the agent is capable of selectively inhibiting TAP activity in stable early endosomes.

5. The agent of claim 4, wherein the agent is a TAP inhibitor coupled to a second agent being capable of directing said TAP inhibitor to stable early endosomes.

6. The agent of claim 5, wherein the TAP inhibitor is US6 and the second agent is transferring or a ligand of the mannose receptor.

7. The agent of any preceding claim, wherein the agent is contained in a pharmaceutical composition comprising the agent and, optionally, one or more pharmaceutically acceptable carriers.

8. The agent of claim 7, wherein said pharmaceutical composition consists of the agent and one or more pharmaceutically acceptable carriers.

9. The agent of any of claims 7 or 8, wherein said treatment consists of administering said pharmaceutical composition.

10. The agent of any preceding claim, wherein the autoimmune disease is an autoimmune disease induced by cross-presentation.

11. The agent of any preceding claim, wherein the autoimmune disease is selected from the group consisting of early onset diabetes mellitus, multiple sclerosis, premature ovarian failure, scleroderma, Sjögren's disease, lupus erythematodes, vitiligo, alopecia, polyglandular failure, Grave's disease, hypothyroidism, polymyosititis, pemphigus, Crohn's disease, colitis ulcerosa, autoimmune hepatitis, hypopituitarism, myocardititis, Addison's disease, uveitis, pernicious anemia, hypoparathyroidism, and rheumatoid arthritis.
